# EUROPEAN PATENT APPLICATION

(11) **EP 0 640 647 A2**
(43) Date of publication of application: **01.03.1995**
(21) Application number: 94112944.7
(22) Date of filing: 18.08.1994
(51) Int. Cl.: C08L 5/08, C08L 5/10, A61K 47/36, A61L 27/00

(54) **Ionically crosslinked glycosaminoglycan gels for soft tissue augmentation and drug delivery**

(30) Priority: 26.08.1993 US 112833
(71) Applicant: COLLAGEN CORPORATION, Palo Alto, California 94303 (US)
(72) Inventor: Berg, Richard A., Los Altos, CA 94024 (US); Rhee, Woonza M., Palo Alto, CA 94306 (US)
(74) Representative: Schwan, Gerhard, Dipl.-Ing.

(57) **Abstract**

The present invention pertains to the use of glycosaminoglycans, chemically derivatized glycosaminoglycans, and optionally, chemically derivatized collagens to form ionically crosslinked gels useful in mammal soft tissue augmentation and in drug delivery systems. The derivatized glycosaminoglycans can be used to form an ionically homogeneous gel comprising one or more species of glycosaminoglycan derivative or can be used to form an ionically crosslinked heterogeneous gel comprising one or more negatively charged species of glycosaminoglycan or collagen derivative in combination with one or more positively charged species of glycosaminoglycan derivative or collagen derivative.

The ionically crosslinked homogeneous or ionically crosslinked heterogeneous gels are produced from liquid solutions which upon adjusting pH *in situ* form a gel.

## Description

### Field of the Invention

This invention relates generally to biocompatible, ionically crosslinked gels which comprise one or more species of glycosaminoglycans and/or derivatives of glycosaminoglycans: 1) ionically homogeneous gels containing one or more similarly charged species of glycosaminoglycan derivative, or 2) ionically heterogeneous gels containing two or more oppositely charged species of glycosaminoglycans, glycosaminoglycan derivatives, and/or collagen derivatives. The ionically crosslinked gels are useful in a number of medical applications, particularly as injectable compositions for soft tissue augmentation or drug delivery.

### Background of the Invention

A variety of different glycosaminoglycans are known. Examples of such include the chondroitin sulfates, keratan sulfate, keratosulfate, heparin, chitin, and hyaluronic acid. Compositions of these glycosaminoglycans are known to be useful in connection with the augmentation of soft tissue. Hyaluronic acid in particular has been used in various compositions for use in soil tissue augmentation. One example of such is disclosed by Balazs et al., Matrix Engineering, Blood Coagulations and Fibrinolysis, Volume 2, pages 173-178, 1991, which discloses chemical modifications of hyaluronic acid, such as preparing hyaluronic acid gels by forming sulphonyl-bis-ethyl crosslinks between hydroxyl groups of the polysaccharide chains. Information regarding how hyaluronic acid and its derivatives interact within a biological system are also known, as disclosed by Balazs et al., Clinical Uses of Hyaluronin, Ciba Foundation Symposium 143, a Wiley-Interscience publication (1989) and by Doillon et al., Fibroblast Growth on a Porous Sponge Containing Hyaluronic Acid and Fibronectin, BioMaterials 8:195-200(1987).

Various biocompatible viscoelastic gel slurries are disclosed within European patent application number 91303606.7, published January 15, 1992, which refers to various types of crosslinked polysaccharides useful in soil tissue augmentation.

U.S. patent 4,378,017, issued March 29, 1983 to Kasugi et al., discloses combining fibrous nonsoluble collagen with deacetylated chitin to form an insoluble material.

U.S. patent 4,448,718, issued May 15, 1984 to Yannas et al., discloses combining collagen with glycosaminoglycan and glutaraldehyde to form a crosslinked composite material.

U.S. patent 4,451,397, issued May 29, 1984 to Huc et al., discloses mixing ground collagenous material, containing native crosslinked collagen in gel form, with glycosaminoglycan to form a gel.

Hyaluronic acid has been specifically examined and discussed in a series of U.S. patents. For example, U.S. patent 4,582,865, issued April 15, 1986, to Balazs et al., discloses crosslinking gels of hyaluronic acid which may be combined with hydrophilic polymers. The basic chemistry behind the gels formed indicates that the hyaluronic acid is reacted with divinyl sulfone to form a crosslinked gel. U.S. patent 5,017,229, issued May 21, 1991, to Burns et al., discloses different types of water-soluble gels formed with hyaluronic acid. Burns et al. teach reacting hyaluronic acid with one or more activating agents such as 1-(3-dimethylaminopropinyl)-3-ethyl-carbodiimide(EDC).

U.S. patent 4,937,270, issued June 26, 1990, to Hamilton et al., also discloses activating hyaluronic acid with an activating agent and thereafter reacting the activated hyaluronic acid with a nucleophile in order to form a water-insoluble biocompatible gel.

U.S. patent 5,099,013, issued March 24, 1992, to Balazs et al., discloses yet another hyaluronic acid containing composition. In accordance with this disclosure, aldehyde crosslinking groups are used to covalently bind hyaluronic acid chains to each other. In a related U.S. patent to Balazs et al., i.e., U.S. patent 5,128,326, issued July 7, 1992, the hyaluronic acid is copolymerized with another hydrophilic polymer using a crosslinking agent in the form of divinyl sulphone.

U.S. patent 5,137,875, issued August 11, 1992 to Tsunenaga et al., discloses combining nonporous particulate collagen with hyaluronic acid.

U.S. patent 5,166,187, issued November 24, 1992 to Collombel et al., discloses collagen that is not ionically modified used in combination with chitosan and a glycosaminoglycan to form an ionic gel.

European patent application number 86303391.6 discloses combining chitosan with collagen.

U.S. patent 5,202,431, issued April 13, 1993, discloses esters of hyaluronic acid wherein a portion of the carboxylic groups on the hyaluronic acid are esterified in order to form gels which are indicated as being useful for soil tissue augmentation.

### Summary of the Invention

In accordance with the present invention, biocompatible gels useful in a variety of medical applications are formed as a result of ionic interactions between positive and negative charges on molecules having a net neutral charge (ionically homogeneous gels), or between a molecule having a net positive charge and one having a net negative charge (ionically heterogeneous gels). The ionically homogeneous gels may contain one or more species of glycosaminoglycan derivative. The ionically heterogeneous gels must contain at least two species selected from glycosaminoglycans, glycosaminoglycan derivatives, and/or collagen derivatives.

To form the ionically homogeneous gels of the present invention, glycosaminoglycans are derivatized by deacetylation (removal of the -COCH₃ group) or desulfation (removal of the -SO₃ group) to provide free amino groups, resulting in glycosaminoglycan derivatives which have a net positive charge at acidic pH (below 6), a net negative charge at basic pH (above 9), and a net neutral charge at neutral pH (above 6 and below 9). However, within the substantially neutral molecule, there are charged groups which are attracted to oppositely charged groups within the same molecule and on other molecules. These ionic interactions within and between molecules cause the formation of a gel at or around neutral pH. Thus, the glycosaminoglycan derivative can be maintained in a liquid state for storage above pH 9, for example; the addition of a small amount of acid (to provide a neutral pH) will cause the formation of the ionically homogeneous gel of the invention. Generally, the pH of the composition will be adjusted to within the range of 6 - 8.5 just prior to administration to human or animal tissue, to avoid tissue damage that may occur at more extreme pH levels. The composition will subsequently form a gel *in situ* as the glycosaminoglycan derivative reaches physiological pH. Glycosaminoglycans which can be derivatized by either deacetylation, desulfation, or both to form species having a net neutral charge include hyaluronic acid, the chondroitin sulfates, chitin, heparin, and combinations thereof.

To form the ionically heterogeneous gels of the invention, glycosaminoglycans, glycosaminoglycan derivatives, and/or collagen derivatives which are positively or negatively charged at neutral pH are reacted with molecules having the opposite charge, resulting in the formation of an ionically crosslinked gel having a net neutral charge. Glycosaminoglycans such as hyaluronic acid, chondroitin sulfate A, chondroitin sulfate C, and keratan sulfate can be derivatized by esterification, then further derivatized by deacetylation and/or desulfation, to have a net positive charge. Positively charged glycosaminoglycan derivatives, such as chitosan or esterified deacetylated hyaluronic acid, can be combined with negatively charged nonderivatized glysoaminoglycans, such as sodium hyaluronate and/or sodium chondroitin sulfate, to form the ionically heterogeneous gels of the invention. Positively charged glycosaminoglycan derivatives and negatively charged nonderivatized glycosaminoglycans can be combined with succinylated collagen (net negative charge) or methylated collagen (net positive charge), respectively, to form gels. The combination of appropriate amounts of negatively charged and positively charged compounds results in formation of a gel having a net neutral charge at neutral pH. The oppositely charged species can be stored separately, and then combined just prior to administration to result *in situ* gel formation.

An important feature of the invention is that the glycosaminoglycans, glycosaminoglycan derivatives, and collagen derivatives are biocompatible and have low immunogenicity, and are therefore suitable for use in a variety of medical applications, particularly soil tissue augmentation and delivery of therapeutic agents.

Another feature of the present invention is that the glycosaminoglycans and glycosaminoglycan derivatives crosslink with each other to form a firm gel without the need for additional crosslinking agents.

An additional feature is that other charged compounds, such as certain collagen derivatives, can be incorporated into the glycosaminoglycan gels to produce compositions having a variety of physical and chemical characteristics.

An advantage of the present invention is that the glycosaminoglycan derivatives can be stored at a pH above 9 or below 6, at which pH the glycosaminoglycan derivatives are in ionic form (net positive or net negative charge) and therefore have a low viscosity. The pH of the ionically homogeneous gels can be adjusted to neutral, or the charged species of the ionically heterogeneous gels can be combined, just prior to administration so that the compositions are still readily injectable into soil tissue, where they will form gels shortly (generally within a few minutes) after administration.

These and other features of the present invention will become apparent to those persons skilled in the art upon reading the details of the structure, synthesis, and usage of the glycosaminoglycan derivatives disclosed and described below.

### Detailed Description of Preferred Embodiments of the Invention

It must be noted that, as used in this specification and the appended claims, the singular forms "a", "an", and "the" include plural referents, unless the context clearly dictates otherwise. Thus, for example, reference to "a hyaluronic acid" includes one or more conjugates, reference to "an implant" includes one or more different types of implants known to those skilled in the art, and reference to "the method" includes different types of methods of the same general type and so forth.

Unless defined otherwise, all technical and scientific terms used herein have the same meaning as commonly understood by one of ordinary skill in the art to which this invention belongs. Although any methods and materials similar or equivalent to those described herein may be useful in the practice or testing of the present invention, only the preferred methods and materials are described below; it is not intended that the invention be limited to these preferred embodiments, however. The invention is intended to have the scope defined by the attached claims.

All publications mentioned herein are incorporated herein by reference. Specific terminology of particular importance to the description of the present invention is defined below.

### Definitions

The term "glycosaminoglycan" is intended to encompass complex polysaccharides (which are not biologically active, i.e., not compounds such as ligands or proteins) having repeating units of either the same saccharide subunit or two different saccharide subunits. Some examples of glycosaminoglycans include dermatan sulfate, hyaluronic acid, the chondroitin sulfates, chitin, keratan sulfate, keratosulfate, heparin, and derivatives thereof. In general, the glycosaminoglycans are extracted from a natural source, purified and derivatized. However, they may be synthetically produced or synthesized by modified microorganisms such as bacteria.

The term "hyaluronic acid" is intended to encompass naturally occurring and synthetic forms of the polymer-(C₈H₁₃O₄N)ₙ·(C₆H₈O₅)ₙO-(n=1 to n=5,000), and derivatives thereof. Particularly preferred derivatives include those having functionalized moieties which allow chemical reaction with another molecule to form a covalent bond, such as deacetylated hyaluronic acid. Hyaluronic acid includes alternating units of 1,4-linked N-acetyglucosamine and glucuronic acid units as shown below.

Hyaluronic acid is a viscous, high molecular weight mucopolysaccharide found in mammalian body fluids and connective tissue. The formula for hyaluronic acid is shown below:

### Hyaluronic Acid

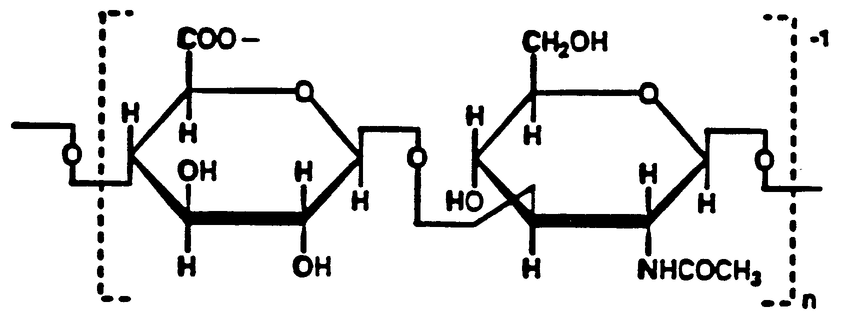

Alternating units of 1,4-linked
N-acetylglucosamine and glucuronic acid
wherein n ranges from 1 to about 5,000.

The term "chondroitin sulfate" as used herein is meant to encompass three major compounds: chondroitin sulfate A, dermatan sulfate (also known as chondroitin sulfate B, which is an isomer of chondroitin sulfate A), and chondroitin sulfate C. The structures of these three compounds are shown below:

### Chondroitin Sulfate A

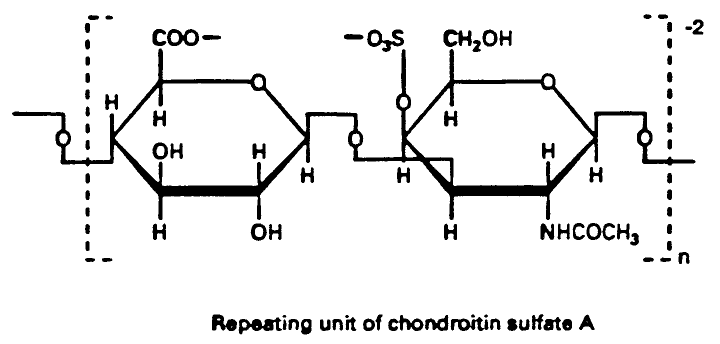

wherein n ranges from about 10 to about 300;

### Chondroitin Sulfate C

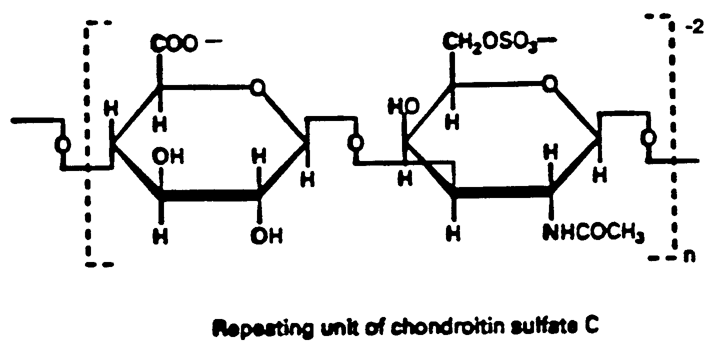

wherein n ranges from about 20 to about 200;

### Dermatan Sulfate

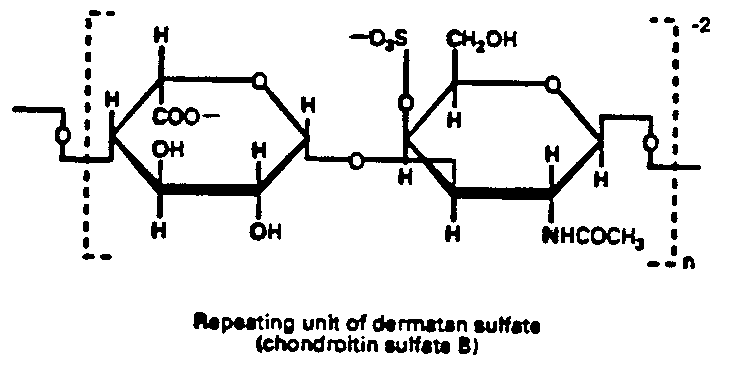

wherein n ranges from about 10 to about 300.

The term "chitin" is intended to encompass polymers comprising repeating units of N-acetylglucosamine. The structure of chitin is shown below:

### Chitin

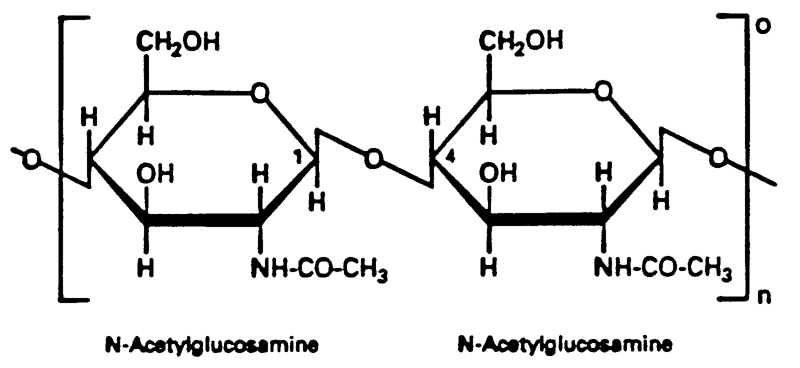

wherein n ranges from about 500 to about 2,000.

The term "chitosans" refers to both partially and fully deacetylated chitins. The term "chitosan 1" refers to partially deacetylated chitin, as shown below:

### Chitosan 1

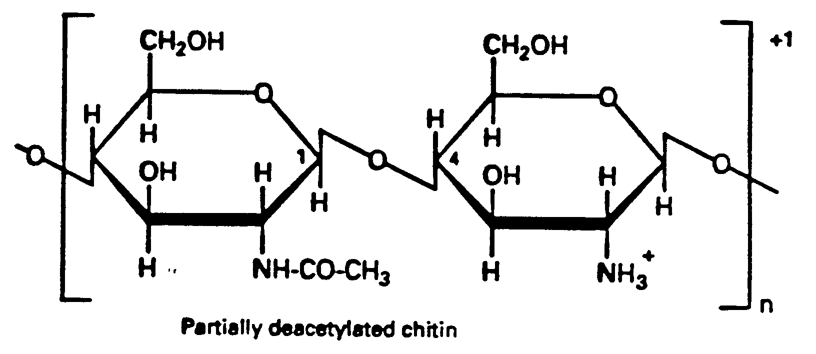

wherein n ranges from about 500 to about 2,000.

The term "chitosan 2" refers to fully deacetylated chitin, as shown below:

### Chitosan 2

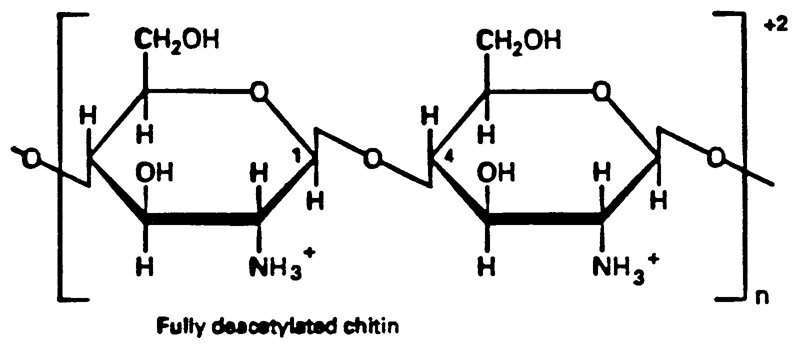

wherein n ranges from about 500 to about 2,000.

The term "keratan sulfate" refers to polymers having the repeating structure shown below:

### Keratan Sulfate

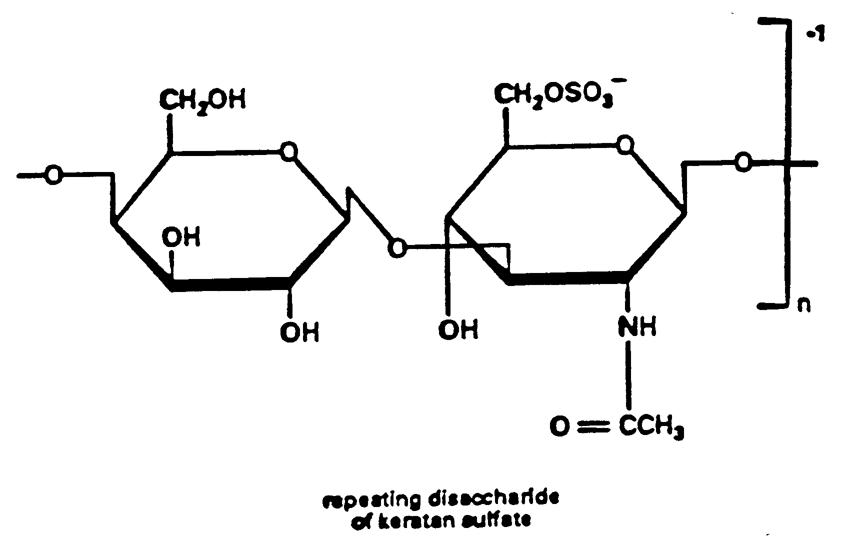

wherein n ranges from about 10 to about 100.

The term "keratosulfate" refers to a polymer that is an isomer of keratan sulfate, having the repeating structure shown below:

### Keratosulfate

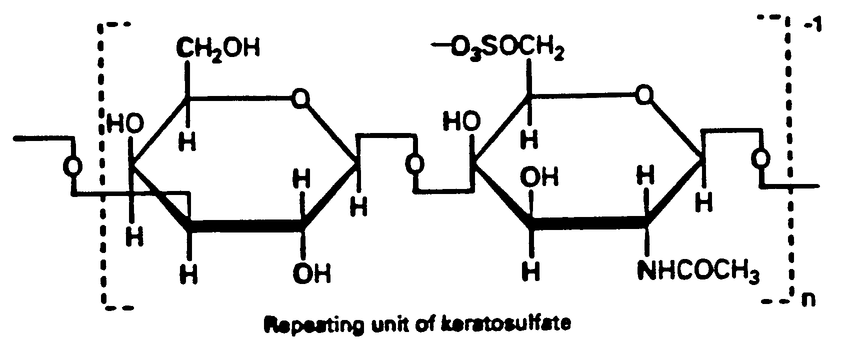

wherein n ranges from about 10 to about 100.

The term "heparin" refers to polymers comprising alternating units of sulfated glucosamine and sulfated glucuronic acid, as shown below.

### Heparin

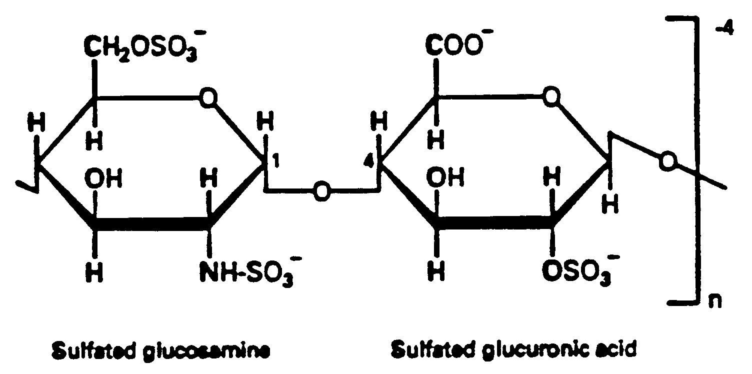

wherein n ranges from about 2 to about 3,000.

The term "collagen" is used in its conventional sense to describe a material which is the major protein component of the extracellular matter of bone, cartilage, skin, and connective tissue in animals and derivatives. Collagen in its native form is typically a rigid, rod-shaped molecule approximately 300 nm long and 1.5 nm in diameter. It is composed of three collagen polypeptides which form a tight triple helix. The collagen polypeptides are characterized by a long midsection having the repeating sequence -Gly-X-Y-, where X and Y are often proline or hydroxyproline, bounded at each end by the "telopeptide" regions, which constitute less than about 5% of the molecule. The telopeptide regions of the collagen chains are typically responsible for the crosslinking between chains, and for immunogenicity of the protein. Collagen occurs in several "types", having differing physical properties. The most abundant types are Types I-III. The term "collagen" as used herein includes these and other known types of collagen including natural collagen and collagen which is processed or modified, i.e., various collagen derivatives. Collagen is typically isolated from natural sources, such as bovine hide, cartilage, or bones. Bones are usually dried, defatted, crushed, and demineralized to extract collagen, while hide and cartilage are usually minced and digested with proteolytic enzymes (other than collagenase). As collagen is resistant to most proteolytic enzymes, this procedure conveniently serves to remove most of the contaminating protein found with collagen.

The term "succinylated collagen" refers to collagen that has been chemically derivatized by the addition of succinic anhydride. A method for preparing succinylated collagen is disclosed in U.S. Patent 4,164,559. Succinylated collagen has a net negative charge.

The term "methylated collagen" refers to collagen that has been chemically derivatized by the addition of methanol. A method for preparing methylated collagen is also disclosed in U.S. Patent 4,164,559. Methylated collagen has a net positive charge.

The terms "treat" and "treatment" as used herein refer to augmentation, repair, prevention, or alleviation of defects, particularly defects due to loss or absence of tissue. Additionally, "treat" and "treatment" also refer to the prevention, maintenance, or alleviation of disorders or disease using a biologically active protein.

The terms "cytokine" and "growth factor" are used to describe biologically active molecules and active peptides (which may be either naturally occurring or synthetic) which aid in healing or regrowth of normal tissue. The function of cytokines and growth factors is twofold: 1) they can incite local cells to produce new collagen or tissue, or 2) they can attract cells to the site in need of correction. As such, cytokines serve to encourage "biological anchoring" of the implant within the host tissue. As previously described, the cytokines and growth factors can be admixed with the glycosaminoglycan gel. For example, one may incorporate cytokines such as interferons (IFN), tumor necrosis factors (TNF), interleukins, colony stimulating factors (CSFs), or growth factors such as osteogenic factor extract (OFE), epidermal growth factor (EGF), transforming growth factor (TGF) alpha, TGF-β (including any combination of TGF-βs), TGF-β1, TGF-β2, platelet derived growth factor (PDGF-AA, PDGF-AB, PDGF-BB), acidic fibroblast growth factor (FGF), basic FGF, connective tissue activating peptides (CTAP), β-thromboglobulin, insulin-like growth factors, erythropoietin (EPO), nerve growth factor (NGF), bone morphogenic protein (BMP), osteogenic factors, and the like. Incorporation of cytokines, growth factors, and appropriate combinations of cytokines and growth factors can facilitate the regrowth and remodeling of the implant into normal tissue.

The term "pharmaceutically active compound" or "pharmaceutically active drug" are used interchangeably herein. The terms refer to drugs or medicines which are conventionally given to patients in order to create a desired pharmacological effect. Such "compounds" or "drugs" include those which are listed and described within the latest edition of the Physicians Desk Reference.

The term "chemotherapeutic agents" refers to alkylating agents such as, but not limited to, nitrogen mustards, ethylenimine derivatives, alkyl sulfonates, nitrosoureas, chlorambucil (Leukeran), busulfan, streptozotocin; antimetabolites such as folic acid analogs (methotrexate), pyrimidine analogs (fluorouracil, cytarabine, azaribine), purine analogs (mercaptopurine, thioguanine); natural products such as vinca alkaloids (vinblastine); and antibiotics such as dactinomycin (actinomycin D), daunarubicin, doxorubicin (adriamycin), and bleomycin.

The term "effective amount" refers to the amount of composition required in order to obtain the effect desired. Thus, a "tissue growth-promoting amount" of a composition containing a cytokine or growth factor refers to the amount of cytokine or growth factor needed in order to stimulate tissue growth to a detectable degree. Tissue, in this context, includes connective tissue, bone, cartilage, epidermis and dermis, blood, and other tissues. The actual amount which is determined to be an effective amount will vary depending on factors such as the size, condition, sex, and age of the patient and can be more readily determined by the caregiver.

The term "sufficient amount" as used herein is applied to the amount of carrier used in combination with the hyaluronic acid formulations of the invention. A sufficient amount of pharmaceutically acceptable carrier is that amount which, when mixed with the hyaluronic acid formulation, renders it in the physical form desired, for example, compositions which can be formed into injectable solutions. The amount of the carrier can be varied and adjusted depending on the particular hyaluronic acid formulation used and the end result desired. Such adjustments will be apparent to those skilled in the art upon reading this disclosure.

The term "*in situ*" as used herein means "at the site of administration." In accordance with the present invention, the hyaluronic acid is subjected to deacetylation. The deacetylated hyaluronic acid is placed in formulation at a pH where the deacetylated hyaluronic acid will not react with itself or "crosslink." However, prior to use, the pH is raised or decreased as needed to a level where crosslinking will occur. Thereafter, in a relatively short period of time, the formulation is injected. The injection is to take place prior to crosslinking (or at least prior to substantial crosslinking resulting in gel formation), so that the material can be easily and smoothly injected beneath the skin for soft tissue augmentation. Once the injected formulation is in place, crosslinking will proceed "*in situ*" and the composition will form a firm gel for soft tissue augmentation.

### General Method

To produce the ionically crosslinked glycosaminoglycan gels of the invention, it is first necessary to obtain one or more species of glycosaminoglycan in purified form. As chemical synthesis is not generally economically efficient, the glycosaminoglycans are generally extracted from animal tissue or bacteria. For example, one method of extracting and recovering a purified form of hyaluronic acid on a commercial scale is disclosed within U.S. patent 4,141,973, which is incorporated herein by reference. This method makes it possible to obtain ultra-pure hyaluronic acid having a protein content less than 0.5% by weight and a molecular weight of more than 1,200,000 by extracting the hyaluronic acid from rooster combs or human umbilical cords. Hyaluronic acid obtained by this methodology is marketed and is commercially available under the trademark HEALON®, available from Pharmacia, Inc. (Piscataway, NJ). Glycosaminaglycans such as the chondroitin sulfates, keratan sulfate, keratosulfate, chitin, chitosan, and heparin can he obained from Sigma Chemical Co. (St. Louis, MO).

The ionically crosslinked glycosaminoglycan gels of the present invention are, preferably, used for soft tissue augmentation or for delivery of various therapeutic agents such as growth factors, cytokines, biologics, and chemotherapeutic agents. The glycosaminoglycans must he obtained in a relatively pure form or a form which can be readily sterilized before being subjected to the methodology of the present invention. Although it is possible to carry out a certain degree of sterilization of a gel after formation, it is preferable to have the starting material sterilized and purified prior to gel formation to the extent that the starting materials are pharmaceutically pure, i.e., they do not contain bacterial contaminants and do not contain significant amounts of other materials which would affect the pharmaceutical properties of the gel compositions. Specifically, it is desirable to remove any substances which would create an immune reaction, as immune reactions are undesirable for compositions used in soft tissue augmentation.

### Deacetylation of Glycosaminoglycans to Provide Free Amino Groups

To form certain of the ionically crosslinked glycosaminoglycan gels of the invention, particularly the ionically homogeneous gels, it is first necessary to chemically derivatize the glycosaminoglycan. Glycosaminoglycans having a -NHCOCH₃ group, such as hyaluronic acid, the chondroitin sulfates, chitin, keratan sulfate, and keratosulfate, can be deacetylated (removal of the -COCH₃ group) by the addition of a strong base such as sodium hydroxide to provide free amino (-NH₂) groups. Deacetylation of hyaluronic acid is shown in Reaction Scheme 1.

### Deacetylation of Hyaluronic Acid

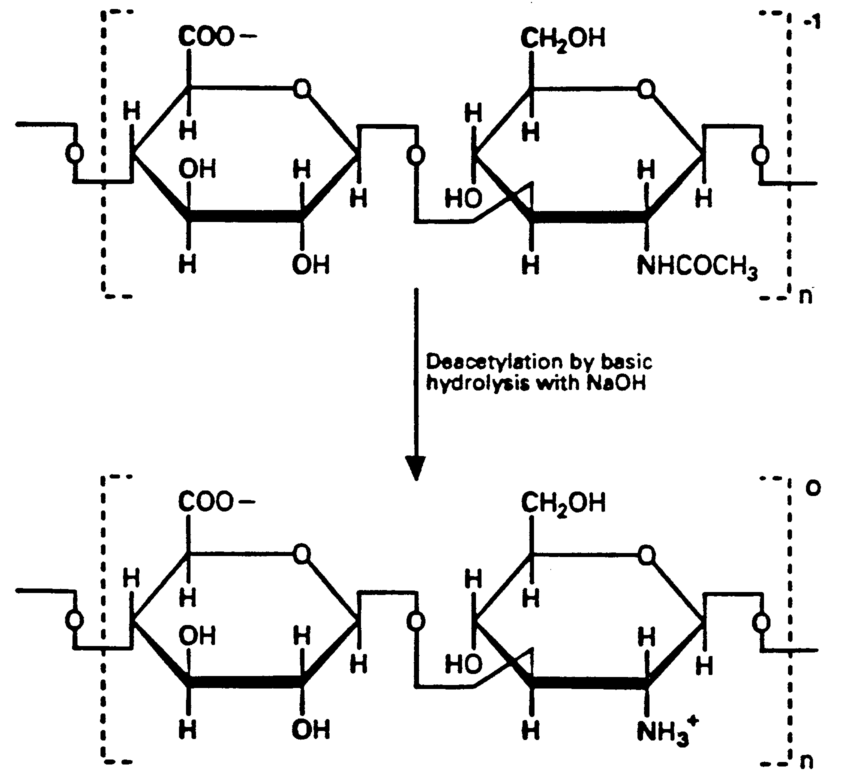

### Reaction Scheme 1

Deacetylated glycosaminoglycans exist in different ionic forms depending on the pH of the surrounding environment, that is, whether the deacetylated glycosaminoglycan is present in a basic, acidic, or neutral solution. The three ionic forms of deacetylated hyaluronic acid are shown below:

### Deacetylated Hyaluronic Acid at Basic pH (pH > 9)

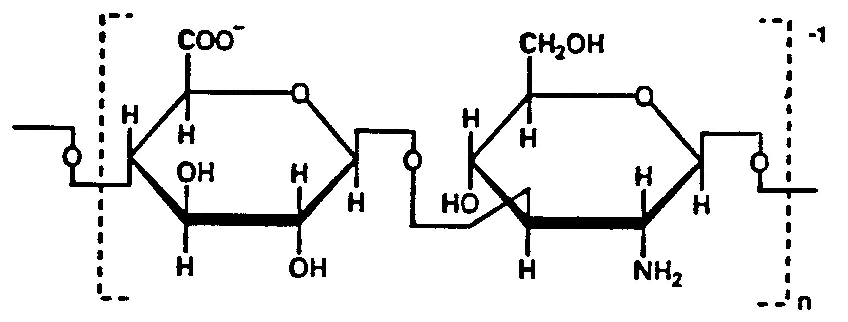

### Deacetylated Hyaluronic Acid at Acidic pH (pH < 6)

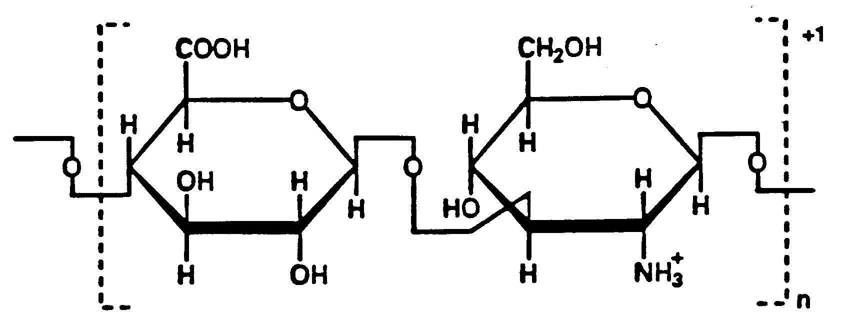

### Deacetylated Hyaluronic Acid at Neutral pH (6 < pH < 9; preferably, pH = 7.2 ± 0.5)

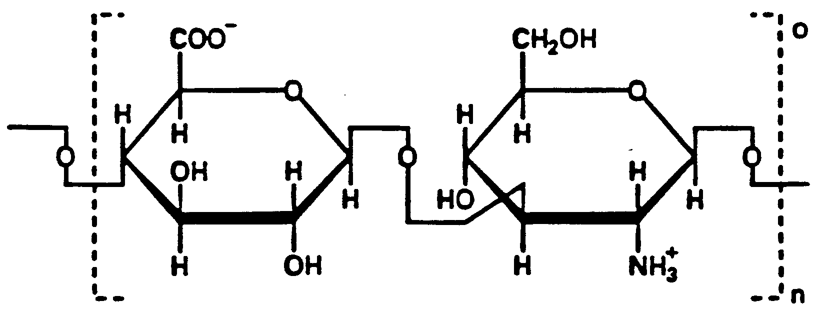

Although neutral pH, as indicated above, falls in the range of 6 to 9, the preferred pH range for the ionically crosslinked gels of the present invention is at physiological pH: (6-8.5) and most preferably about 7.2 ± 0.5. As shown above, when deacetylated hyaluronic acid is at basic pH, the carboxyl group (-COOH) is deprotonated to -COO⁻; at acidic pH, the amino group (-NH₂) is protonated to -NH₃⁺. However, when deacetylated hyaluronic acid is maintained at a relatively neutral pH, both situations are in effect within the same molecule: the carboxyl group is deprotonated and the amino group is protonated. Although the molecule has a net neutral charge at neutral pH, positive and negative charges within molecules allow for significant ionic interaction between chains (and within the same chain for long-chain polymers), providing ionic crosslinking and resulting in the formation of a relatively firm, ionically homogeneous gel.

Deacetylation of chondroitin sulfate C, shown in Reaction Scheme 2, is accomplished in a similar manner to that described for hyaluronic acid.

### Deacetylation of Chondroitin Sulfate C

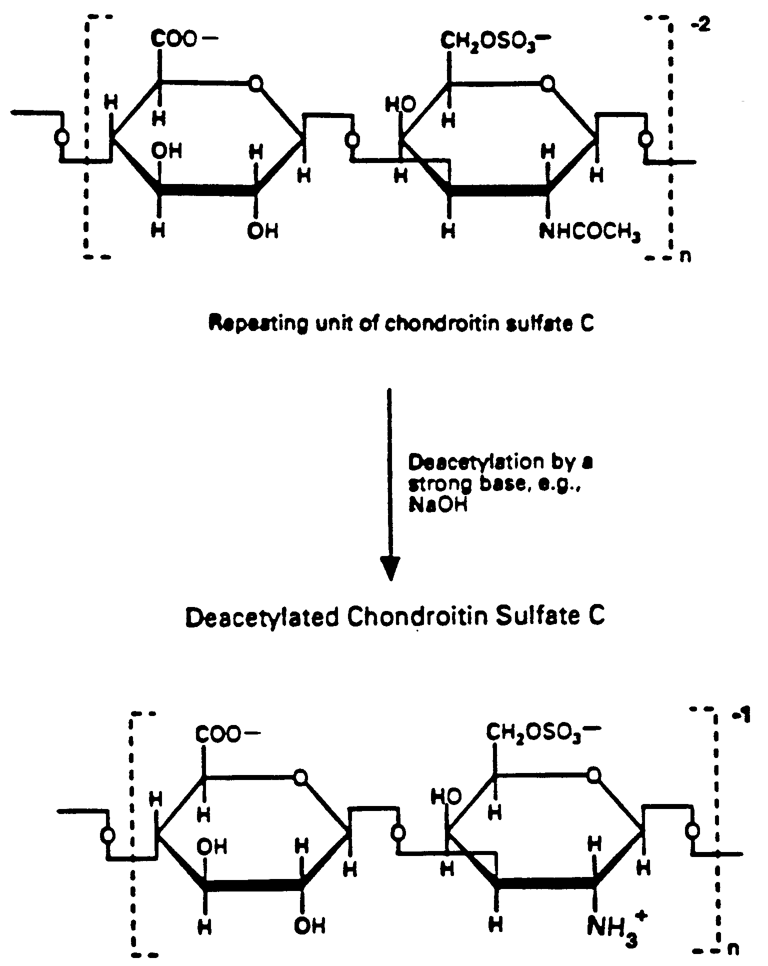

### Reaction Scheme 2

Deacetylation of chondroitin sulfate A, dermatan sulfate, keratan sulfate, and keratosulfate is effected in a similar manner to that shown above for hyaluronic acid and chondroitin sulfate C.

Chitin can be partially deacetylated to form chitosan 1 (one free amino group) or fully deacetylated to form chitosan 2 (two free amino groups), as shown in Reaction Scheme 3. The degree of deacetylation can be controlled by adjusting the relative amount of base reacted with the chitin.

### Partial Deacetylation of Chitin with NaOH to Yield Chitosan 1

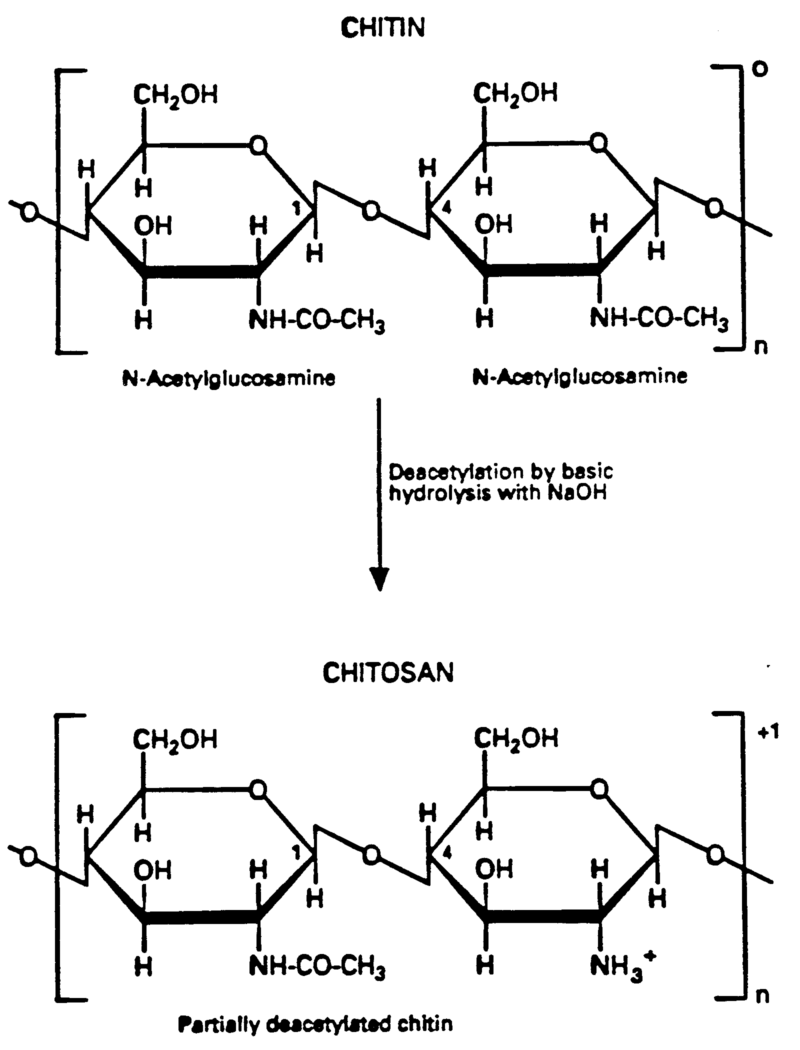

or

### Full Deacetylation of Chitin with NaOH to Yield Chitosan 2

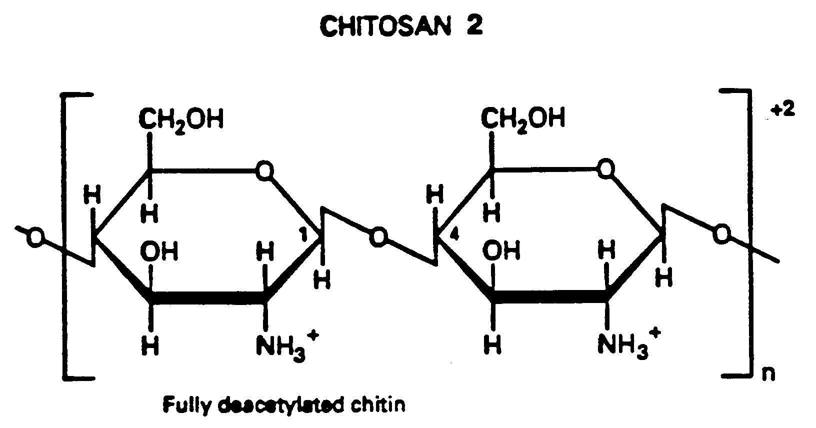

### Reaction Scheme 3

As shown above, each disaccharide of chitosan 1 has a +1 charge at pH 7; chitosan 2 has a +2 charge. Either of these species could be combined with an appropriate amount of a negatively charged nonderivatized glycosaminoglycan or collagen derivative to form an ionically heterogeneous gel of the invention.

### Desulfation of Glycosaminoglycans to Provide Active Amino Groups

Glycosaminoglycans having a sulfate (-SO₃) group, such as heparin, the chondroitin sulfates, keratan sulfate, and keratosulfate, can be desulfated by the addition of a strong base such as sodium hydroxide to provide free amino group (-NH₂), or ionically neutral groups such as hydroxyl (-OH) groups. Desulfation of heparin is shown in Reaction Scheme 4.

### Desulfation of Heparin

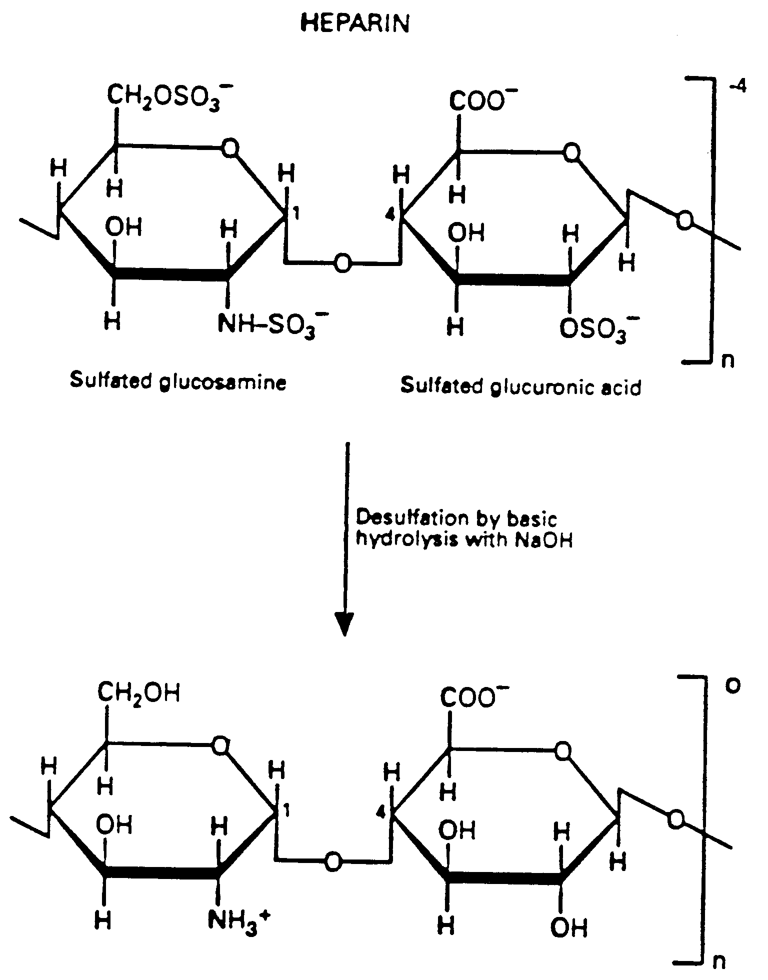

### Desulfated Heparin

### Reaction Scheme 4

Because it contains both free amino and free carboxyl groups, desulfated heparin exists in similar ionic forms as deacetylated hyaluronic acid, as shown below.

### Desulfated Heparin at Basic pH (pH > 9)

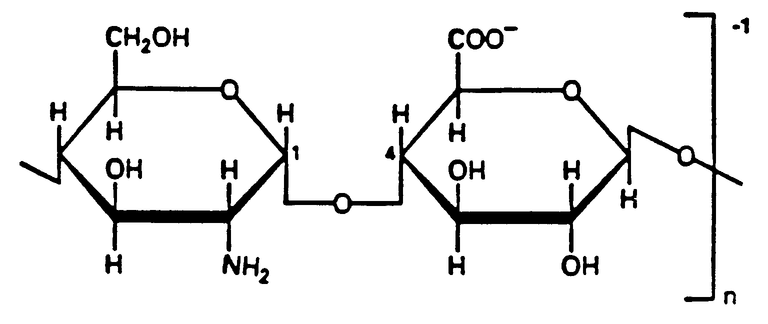

### Desulfated Heparin at Acidic pH (pH < 4)

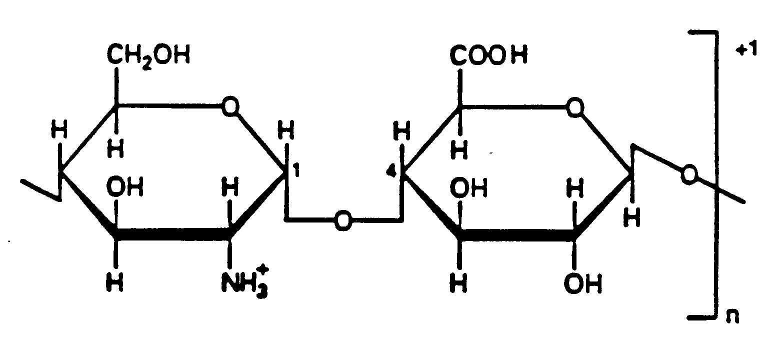

### Desulfated Heparin at Neutral pH (6 < pH < 9, preferably, pH = 7.2 ± 0.5)

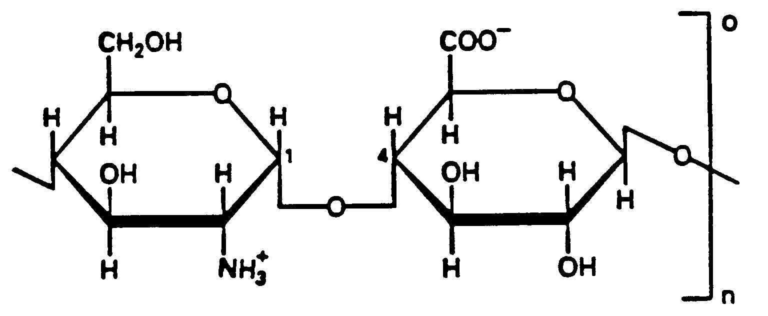

Approaching neutral pH, both positive and negative charges exist within the desulfated heparin molecule, resulting in a net neutral charge for the molecule as a whole. Ionic interactions within and between molecules lead to the formation of a relatively firm, ionically homogeneous gel.

Glycosaminoglycans having both -SO₃ and -NHCOCH₃ groups, such as the chondroitin sulfates, keratan sulfate, and keratosulfate, are subject to both deacetylation and desulfation upon addition of bases such as sodium hydroxide. Desulfation and deacetylation of the chondroitin sulfates creates one free amino group and one ionically neutral group (-OH or -CH₂OH) per disaccharide. The chondroitin sulfates also have one free carboxyl group per dissacharide; therefore, molecules of deacetylated, desulfated chondroitin sulfate have a net neutral charge at or around neutral pH, due to the balance of positive and negative charges within each disaccaride.

As with the chondroitin sulfates, desulfation and deacetylation of keratan sulfate or keratosulfate creates one free amino group and one ionically neutral group (-CH₂OH) per disaccharide. However, because neither keratan sulfate nor keratosulfate contain the balancing carboxyl group, following deacetylation and desulfation, keratan sulfate and keratosulfate each have a net +1 charge per disaccharide at neutral pH (similar to chitosan 1). Deacetylated, desulfated keratan sulfate or keratosulfate could therefore be combined with an appropriate amount of a negatively charged nonderivatized glycosaminoglycan or collagen derivative to form an ionically heterogeneous gel.

### Esterification of Glycosaminoglycans

Glycosaminoglycans having free carboxyl (-COOH) groups, such as hyaluronic acid, chondroitin sulfates A and C, and heparin, can be esterified by the addition of an alcohol (R-OH) such as methanol, ethanol, benzyl alcohol, n-propyl alcohol, n-butyl alcohol, n-phenyl alcohol, or isopentyl alcohol. Esterification of chondroitin sulfate A is shown in Reaction Scheme 5.

### Esterification of Chondroitin Sulfate A

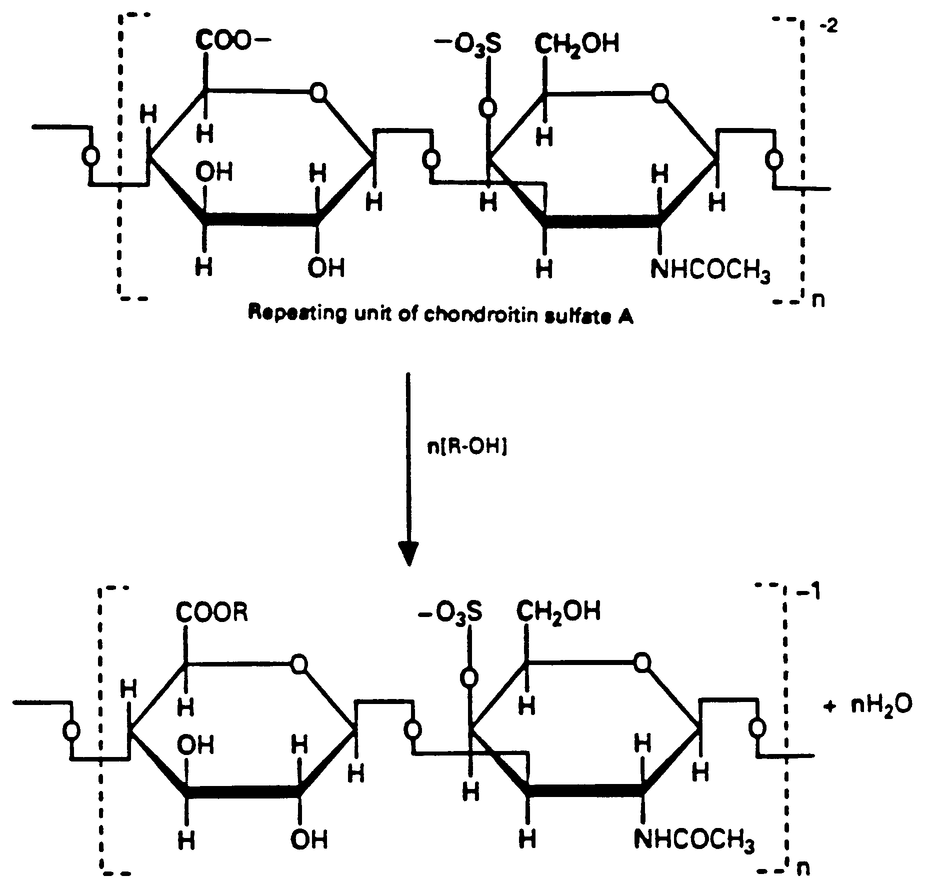

### Esterified chondroitin sulfate A

### Reaction Scheme 5

wherein R is CH₃-, CH₃CH₂-, CH₃CH₂CH₂-, (CH₃)₂CH-, (CH₃)₃C-, or CH₃-(CH₂)₃-.

Esterified glycosaminoglycans can additionally be deacetylated (*e*.*g*., hyaluronic acid) or desulfated (*e*.*g*., heparin) by the addition of sodium hydroxide, resulting in a species that has a net +1 charge per disaccharide. The positively charged glycosaminoglycan derivatives can be combined with negatively charged species to form ionically heterogeneous gels.

As shown in Table 1, chondroitin sulfates A and C can be derivatized by all three methods (deacetylation, desulfation, and esterification).

**Table 1**

| Derivatization of Glycosaminoglycans by Various Methods | | | |
|---|---|---|---|
| Compound | Deacetylation | Desulfation | Esterification |
| Chitin | Yes | No | No |
| Chondroitin sulfate A | Yes | Yes | Yes |
| Chondroitin sulfate B | Yes | Yes | No |
| Chondroitin sulfate C | Yes | Yes | Yes |
| Heparin | No | Yes | Yes |
| Hyaluronic acid | Yes | No | Yes |
| Keratan sulfate | Yes | Yes | No |
| Keratosulfate | Yes | Yes | No |

Reaction Scheme 6 depicts esterification of chondroitin sulfate C by addition of alcohol, followed by deacetylation and desulfation by addition of an appropriate amount of sodium hydroxide.

### Esterification, Deacetylation, and Desulfation of Chondroitin Sulfate C

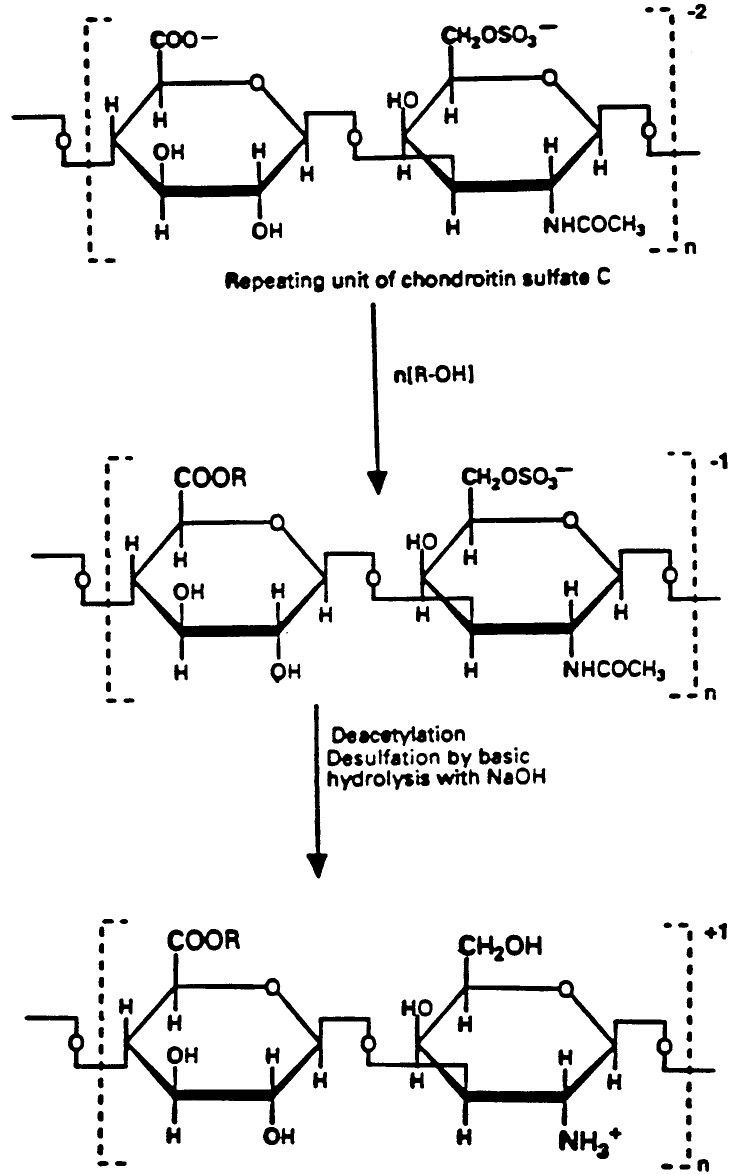

### Reaction Scheme 6

Ionic forms of esterified, deacetylated, desulfated chondroitin sulfate C are shown below.

### Esterified, Deacetylated, Desulfated Chondroitin Sulfate C at Neutral and Acidic pH (pH < 4)

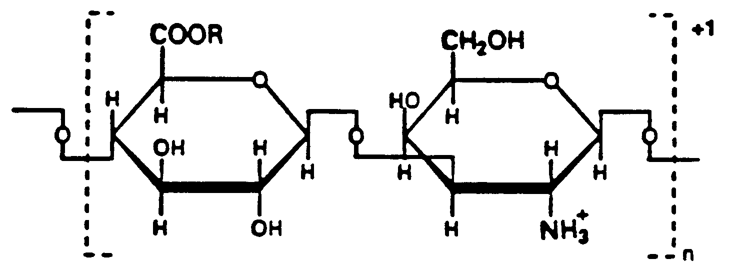

### Esterified, Deacetylated, Desulfated Chondroitin Sulfate C at Basic pH (pH > 9)

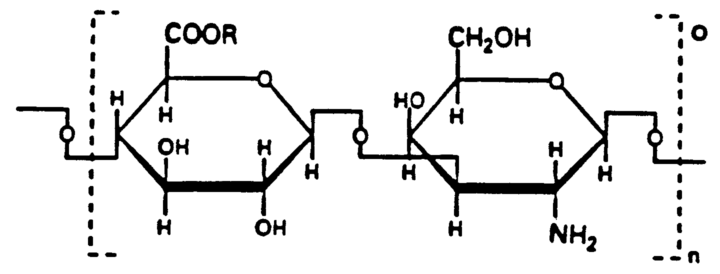

As shown above, due to the conversion of the free carboxyl group to an ionically neutral -COOR group, chondroitin sulfate C which has been derivatized by all three methods has a net charge of +1 per disaccharide at neutral pH.

### Preparation of Ionically Homogeneous Glycosaminoglycan Gels

Preparation of the ionically homogeneous gels of the invention requires one or more species of glycosaminoglycan derivative that has a net neutral charge at neutral pH (balancing negative and positive charges within the same molecule). Glycosaminoglycan derivatives which can be used to prepare the ionically homogeneous gels of the invention include, but are not limited to, the following:
Deacetylated hyaluronic acid
Deacetylated, desulfated chondroitin sulfates A and C
Deacetylated, desulfated dermatan sulfate
Desulfated heparin
The gel may comprise one or more species selected from, but not limited to, the above list.

Following deacetylation or desulfation by addition of sodium hydroxide, the glycosaminoglycan derivatives are generally in an environment having a pH of about 13. The glycosaminoglycan derivatives can be maintained at this high pH for storage because the molecules are in ionic form (net negative charge) and, thus, the composition is in a liquid, flowable state. Any ionic interactions between or within the similarly charged molecules at basic pH are too weak to allow the formation of a stable, ionically crosslinked gel.

The composition will be maintained in a liquid, flowable state as long as the pH of the composition is maintained above 9. Because the administration of compositions having an acidic or basic pH could result in tissue damage, a weak acid is generally added to the composition just prior to administration to decrease the pH to between 6 and 8.5. At this point, the composition can be injected from a relatively fine gauge needle, such as a needle having a gauge in the range of about 20 to about 32 gauge, which has an inner diameter of about 0.90 mm to about 0.23 mm. Accordingly, the derivatized glycosaminoglycan compositions can be injected into relatively sensitive soft tissue areas, such as areas on the face, including areas around the eyes where skin is particularly thin, for the purpose of augmenting the underlying tissue to reduce and/or eliminate fine lines and deeper wrinkles.

Because the addition of acid will cause ionic interactions between the derivatized glycosaminoglycan molecules, causing gel formation to begin, care must be taken to add the acid within a very short period of time (a few minutes) prior to injection to ensure that gel formation occurs within the body and not in the syringe. The composition and a solution of weak acid can he held in the separate barrels of a double-barreled syringe. Upon administration, the glycosaminoglycan derivative will mix with the acid and begin the process of pH neutralization and gel formation just prior to the material being extruded through the needle.

Following administration to the desired tissue site, as the composition reaches physiological pH (approximately 7.2 ± 0.5), the glycosaminoglycan derivatives will ionically interact with one another to form a firm gel *in situ*.

### Preparation of Ionically Heterogeneous Glycosaminoglycan Gels

Preparation of the ionically heterogeneous gels of the invention requires two or more species of glycosaminoglycans, glycosaminoglycan derivatives, or collagen derivatives having opposite charges at neutral pH. Charged species are combined with one or more oppositely charged species, balancing the charges and forming ionically heterogeneous gels. Listed on the following page are examples of species having a net negative and positive charge, respectively, at pH 7:

### Negatively Charged Species (charge per disaccharide where applicable)

Sodium hyaluronate (-1)
Keratan sulfate (-1)
Keratosulfate (-1)
Sodium chondroitin sulfates A and C (-2)
Sodium dermatan sulfate (-2)
Heparin (-4)
Succinylated collagen

### Positively Charged Species (charge per disaccharide where applicable)

Esterified, deacetylated hyaluronic acid (+1)
Esterified, deacetylated, desulfated chondroitin sulfates A and C (+1)
Deacetylated, desulfated keratan sulfate (+1)
Deacetylated, desulfated keratosulfate (+1)
Esterified, desulfated heparin (+1)
Chitosan 1 (+1)
Chitosan 2 (+2)
Methylated collagen
To form the ionically heterogeneous gels of the invention, two or more species selected from, but not limited to, the above list are combined at a neutral pH, preferably ranging from about 6 to about 8.5.

The oppositely charged components of the heterogeneous gels must be stored separately to avoid ionic interactions and subsequent gel formation. The two species may be stored in separate barrels of a double-barreled syringe; if more than two species are being used, species having the same charge may be stored in the same syringe barrel. The oppositely charged species will mix with each other in the syringe just prior to extrusion from the needle, allowing gel formation to occur *in situ*.. By preventing gel formation from beginning until just prior to extrusion, a relatively small gauge needle (approximately 20 to 32 gauge) can be used to administer the ionically heterogeneous compositions.

### Ionically Crosslinked Glycosaminoglycan Gels with Therapeutic Agents

Compositions may be formulated with various therapeutic agents, such as cytokines, growth factors, chemotherapeutic agents, biologics, or antibiotics, by mixing an appropriate amount of the agent into the glycosaminoglycan composition, or by incorporating the agent into the glycosaminoglycan formulation prior to gel formation. Cytokines or growth factors such as TGF-B, are preferably provided at a concentration of about 1 µg/mL to about 5 mg/mL, while the glycosaminoglycan derivative is preferably added to a final concentration providing a 30 to 50-fold molar excess. The relative concentration of chemotherapeutic agents, biologics, or antibiotics included in the glycosaminoglycan formulation must be determined for the specific agent and therapeutic application.

By forming the glycosaminoglycan gel around the therapeutic agent, the gel serves as a depot for localized delivery of the agent. Agents existing in ionic forms may further be held in place by ionic interactions with the glycosaminoglycan gel. The ionic bonds of the gel periodically separate and reform providing a mechanism for the sustained release of a pharmaceutically active agent dispersed in the gel.

### Use and Administration

The ionically crosslinked glycosaminoglycan formulations of the present invention have a variety of uses. A primary use is for soft tissue augmentation. The derivatized glycosaminoglycan solution can be injected into the dermis at a site in need of contour correction, where it will form a gel *in situ* as the composition reaches physiological pH. The composition will remain as a gel due to the ionic interactions between glycosaminoglycan molecules maintained in a neutral pH environment. Further, cytokines or growth factors can be incorporated into the glycosaminoglycan solution prior to injection to aid in "anchoring" the implant in place, i.e., promoting growth between the implant and surrounding tissue. The cytokines and growth factors may be admixed with the glycosaminoglycan composition and may be linked to the glycosaminoglycan molecules via ionic interactions. Formation of the glycosaminoglycan gel *in situ* will hold the cytokines or growth factors in place, preventing them from migrating away from the site in need of augmentation. While in place, the growth factors and cytokines will serve to attract new cells such as fibroblasts to the area and incite local cells to produce new collagen for further tissue augmentation.

The formulations of the invention can also be incorporated into injectable compositions for augmentation of sphincters within the body, such as the urinary, anal, or lower esophageal sphincters. As described above, the incorporation of growth factors and cytokines into the compositions are beneficial in this application, as well as for dermal contour correction.

The glycosaminoglycan gels of the invention have a number of other uses. Because of their lack of immunogenicity, the gels are ideal for use in therapeutic applications where immunological reactions or blood coagulation are to be avoided, such as in dermal wound healing (to avoid scar formation) and cardiovascular applications.

Other uses include ophthalmic applications, such as vitreous gel replacement during cataract surgery, or as an injectable drug delivery system or surgical or dermal dressing. An injectable drug delivery system can be used to deliver a variety of growth factors, drugs, or biologics to localized areas of the body; for example, the localized delivery of anti-cancer drugs to the site of a tumor. The gels may also be used as scaffolding for cartilage replacement. Additionally, the gels could be used in implant fixation in orthopedic surgery or dentistry.

### EXAMPLES

The following examples are put forth so as to provide those of ordinary skill in the art with a complete disclosure and description of how to make the ionically crosslinked glycosaminoglycan gels of the invention and are not intended to limit the scope of the invention. Efforts have been made to ensure accuracy with respect to numbers used (e.g., amounts, temperature, molecular weight, etc.), but there may be minor deviations within experimental error. Unless indicated otherwise, parts are parts by weight, molecular weight is average molecular weight, temperature is in degrees Centigrade, and pressure is at or near atmospheric.

### Example 1

### Ionically Homogeneous Glycosaminoglycan Gels

To produce the inoic crosslinked hyaluronic acid gel of the present invention, 0.79 g of hyaluronic acid was added to 10 ml of a 0.2 M NaOH solution (pH 13). The resulting hyaluronic acid/NaOH solution was allowed to incubate at 20°C for approximately 2 hours to effect partial deacetylation.

Sixty (60) µl of 1 M HCl was added to the deacetylated hyaluronic acid to lower the solution pH to approximately 6. Immediately upon mixing of the hydrochloric acid and deacetylated hyaluronic acid, a firm gel was formed.

### Example 2

### Ionically Heterogeneous Glycosaminoglycan Gels

0.3 ml of sodium hyaluronate (1.5% w/v in PBS, obtained from LifeCore Biomedical) was mixed with 0.2 ml of chitosan (fully deacetylated chitin, 2.5% w/v in PBS, obtained from Sigma). Within approximately five minutes, a gel had formed at pH 7.

The reactants are shown in Reaction Scheme 7, below:

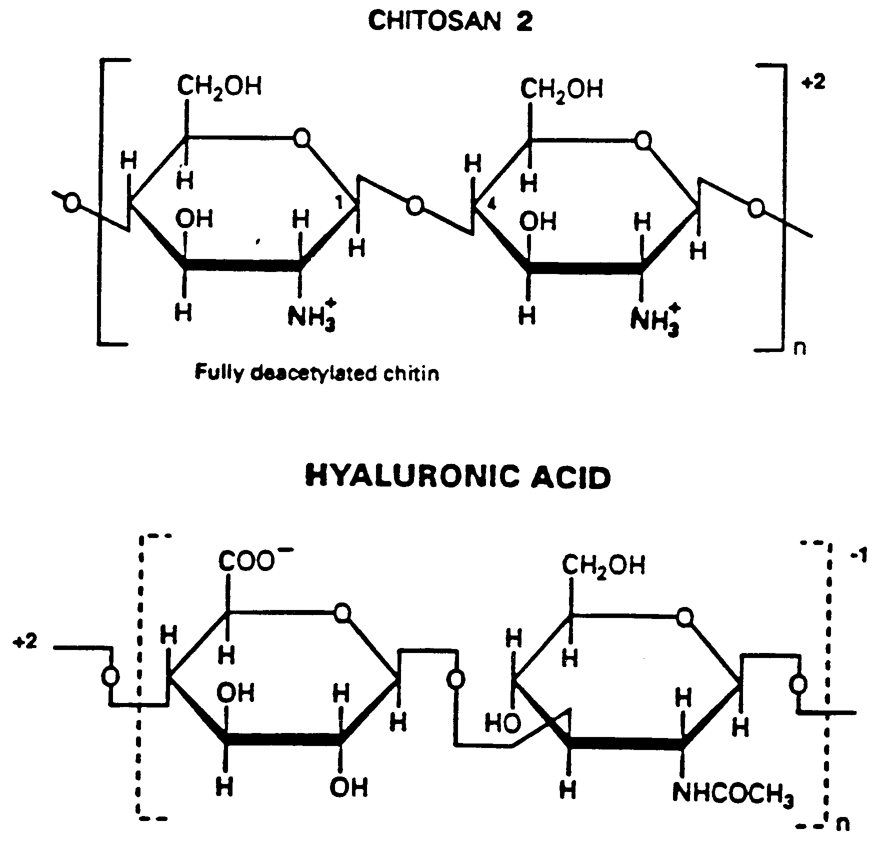

### Reaction Scheme 7

### Example 3

### Ionically Heterogeneous Glycosaminoglycan - Collagen Gels

0.79 grams of air-dried collagen was methylated by immersion in 600 ml of dehydrated methanol containing 0.1 N HCl for two days at room temperature in a tightly sealed vessel. The resulting methylated collagen was dried in a vacuum and suspended in 60 ml of 0.02 M phosphate buffer at pH 11. The pellet was washed three times with 100 ml of water each, then concentrated to have a final protein concentration of 2% (w/v).

Four (4) milliliters of 20 mg/ml methylated collagen was mixed with 2 ml of 15 mg/ml sodium hyaluronate (obtained from LifeCore Biomedical) to form an ionic gel. The melting temperature of the gel was measured using differential scanning calorimetry (DSC) and compared with that of methylated collagen alone. The DSC results are shown in Table 1, below.

DSC is a measure of gel stability which is commonly used to evaluate strength of crosslinking. As shown below, the melting temperature of the ionic gel was approximately 10 degrees higher than that for methylated collagen alone, indicating increased stability of the ionic gel.

**TABLE 1**

| | Melting Temperature (°C, by DSC) |
|---|---|
| Methylated collagen - hyaluronic acid gel | 49.1 |
| Methylated collagen | 38.6 |

The invention is shown and described herein at what is considered to be the most practical, and preferred, embodiments. It is recognized, however, that departures may be made therefrom which are within the scope of the invention, and that obvious modifications will occur to one skilled in the art upon reading this disclosure.

## Claims

1. An ionically homogeneous gel comprising one or more species of glycosaminoglycan derivative having a net neutral charge within the pH range of between about 6 and about 9.

2. The gel of claim 1, wherein the glycosaminoglycan derivative contains free carboxyl and amino groups capable of ionic interaction.

3. The gel of claim 1, wherein the glycosaminoglycan derivative is selected from the group consisting of deacetylated hyaluronic acid, deacetylated desulfated chondroitin sulfate A, deacetylated desulfated dermatan sulfate, deacetylated desulfated chondroitin sulfate C, desulfated heparin, and combinations thereof.

4. The gel of claim 3, wherein the glycosaminoglycan derivative is deacetylated hyaluronic acid.

5. The gel of claim 1, further comprising a therapeutically effective amount of a pharmaceutically active agent selected from the group consisting of a cytokine, a growth factor, a chemotherapeutic agent, and an antibiotic.

6. An ionically heterogeneous gel comprising one or more negatively charged species of glycosaminoglycan and one or more positively charged species of glycosaminoglycan derivative, wherein the negatively charged species and the positively charged species interact to form a gel having a net neutral charge within the pH range of between about 6 and about 9.

7. The gel of claim 6, wherein the negatively charged species of glycosaminoglycan is selected from the group consisting of sodium hyaluronate, keratan sulfate, keratosulfate, sodium chondroitin sulfate A, sodium dermatan sulfate B, sodium chondroitin sulfate C, heparin, and combinations thereof.

8. The gel of claim 6, wherein the positively charged species of glycosaminoglycan derivative is selected from the group consisting of esterified deacetylated hyaluronic acid, esterified deacetylated desulfated chondroitin sulfate A, esterified deacetylated desulfated chondroitin sulfate C, deacetylated desulfated keratan sulfate, deacetylated desulfated keratosulfate, esterified desulfated heparin, chitosan 1, chitosan 2, and combinations thereof.

9. The gel of claim 6, wherein the negatively charged species of glycosaminoglycan is sodium hyaluronate and the positively charged species of glycosaminoglycan derivative is selected from the group consisting of chitosan 1 and chitosan 2.

10. The gel of claim 6, further comprising a therapeutically effective amount of a pharmaceutically active agent selected from the group consisting of a cytokine, a growth factor, a chemotherapeutic agent, and an antibiotic.

11. An ionically heterogeneous gel comprising one or more species of glycosamino glycan derivative and one or more species of collagen derivative, wherein the gel was formed by reaction of a negatively charged glycosaminoglycan species with a positively charged collagen derivative within the pH range of between about 6 and about 9 to form a gel.

12. The gel of claim 11, wherein the negatively charged species of glycosaminoglycan is selected from the group consisting of sodium hyaluronate, keratan sulfate, keratosulfate, sodium chondroitin sulfate A, sodium dermatan sulfate B, sodium chondroitin sulfate C, heparin, and combinations thereof.

13. The gel of claim 11, wherein the positively charged species of collagen derivative is methylated collagen.

14. The gel of claim 13, wherein the negatively charged species of glycosaminoglycan is sodium hyaluronate.

15. The gel of claim 11, further comprising a therapeutically effective amount of a pharmaceutically active agent selected from the group consisting of a cytokine, a growth factor, a chemotherapeutic agent, and an antibiotic.

16. An ionically heterogeneous gel comprising one or more species of glycosaminoglycan derivative and one or more species of collagen derivative, wherein the gel was formed by reaction of a positively charged glycosaminoglycan derivative with a negatively charged collagen derivative within the pH range of between about 6 and about 9 to form a gel.

17. The gel of claim 16, wherein the positively charged species of glycosaminoglycan derivative is selected from the group consisting of esterified deacetylated hyaluronic acid, esterified deacetylated desulfated chondroitin sulfate A, esterified deacetylated desulfated chondroitin sulfate C, deacetylated desulfated keratan sulfate, deacetylated desulfated keratosulfate, esterified desulfated heparin chitosan 1, chitosan 2, and combinations thereof.

18. The gel of claim 16, wherein the negatively charged species of collagen derivative is succinylated collagen.

19. The gel of claim 18, wherein the positively charged species of glycosaminoglycan derivative is selected from the group consisting of chitosan 1 and chitosan 2.

20. A method of preparing an ionically homogeneous glycosaminoglycan gel comprising the steps of:
chemically derivatizing a solution comprising one or more species of glycosaminoglycan to produce an ionically homogeneous solution of one or more charged species; and
affecting the pH of the solution so that the derivatized glycosaminoglycan species ionically interact to form a gel.

21. The method of claim 20, wherein the glycosaminoglycan is chemically derivatized by a method selected from the group consisting of deacetylation, desulfation, and combinations thereof.

22. The method of claim 21, wherein the chemical derivatization is effected by combining the glycosaminoglycan solution with a strong basic solution.

23. The method of claim 22, wherein the strong basic solution comprises a sodium hydroxide solution.

24. The method of claim 20, wherein the glycosaminoglycan is selected from the group consisting of sodium hyaluronate, sodium chondroitin sulfate A, sodium dermatan sulfate, sodium chondroitin sulfate C, heparin, and combinations thereof.

25. A method of preparing an ionically heterogeneous gel comprising the steps of:
providing a first solution comprising one or more negatively charged species of glycosaminoglycan or collagen derivative;
providing a second solution comprising one or more positively charged species of glycosaminoglycan derivative or collagen derivative;
combining the first solution and the second solution; and
allowing the negatively charged species in the first solution and the positively charged species in the second solution to ionically interact to form a gel having a net neutral charge.

26. The method of claim 25, wherein the negatively charged species of glycosaminoglycan or glycosaminoglycan derivative is selected from the group consisting of sodium hyaluronate, keratan sulfate, keratosulfate, sodium chondroitin sulfate A, sodium dermatan sulfate B, sodium chondroitin sulfate C, heparin, esterified chondroitin sulfate C, esterified heparin, and combinations thereof.

27. The method of claim 25, wherein the positively charged species of glycosaminoglycan derivative is selected from the group consisting of esterified deacetylated hyaluronic acid, esterified deacetylated desulfated chondroitin sulfate A, esterified deacetylated desulfated chondroitin sulfate C, deacetylated desulfated keratan sulfate deacetylated desulfated keratosulfate, esterified desulfated heparin, chitosan 1, chitosan 2, and combinations thereof.

28. The method of claim 25, wherein the negatively charged species of glycosaminoglycan is sodium hyaluronate and the positively charged species of glycosaminoglycan derivative is selected from the group consisting of chitosan 1 and chitosan 2.

29. The method of claim 25, wherein the negatively charged species of collagen derivative is succinylated collagen.

30. The method of claim 29, wherein the positively charged species of glycosaminoglycan derivative is selected from the group consisting of chitosan 1 and chitosan 2.

31. The method of claim 25, wherein the positively charged species of collagen derivative is methylated collagen.

32. The method of claim 31, wherein the negatively charged species of glycosaminoglycan is sodium hyaluronate.

33. A method of augmenting soft tissue in a mammal, comprising:
injecting into a site in need of augmentation a liquid composition comprising one or more species of glycosaminoglycan derivative which exhibits both positive and negative charges at a pH within a range from about 6 to about 8.5; and
permitting the liquid composition to form a gel *in situ* by adjustment of the liquid composition pH to within the pH range from about 6 to about 8.5.

34. The method of claim 33, wherein the glycosaminoglycan derivative is selected from the group consisting of deacetylated hyaluronic acid, deacetylated desulfated chondroitin sulfates A, deacetylated desulfated dermatan sulfate, deacetylated desulfated chondroitin sulfate C, desulfated heparin, and combinations thereof.

35. The method of claim 33, wherein the glycosaminoglycan derivative is deacetylated hyaluronic acid.

36. A method of augmenting soft tissue in a meal comprising:
injecting to a site in need of augmentation a liquid composition comprising one or more negatively charged species of glycosaminoglycan, glycosaminoglycan derivative or collagen derivative, and one or more positively charged species of glycosaminoglycan derivative or collagen derivative; and
allowing the negatively charged species and the positively charged species to ionically interact to form a gel *in situ*.

37. The method of claim 36, wherein the negatively charged species of glycosaminoglycan or glycosaminoglycan derivative is selected from the group consisting of sodium hyaluronate, keratan sulfate, keratosulfate, sodium chondroitin sulfate A, sodium dermatan sulfate B, sodium chondroitin sulfate C, heparin, esterified chondroitin sulfate C, esterified heparin, and combinations thereof.

38. The method of claim 36, wherein the positively charged species of glycosaminoglycan derivative is selected from the group consisting of esterified deacetylated hyaluronic acid, esterified deacetylated desulfated chondroitin sulfate A, esterified deacetylated desulfated chondroitin sulfate C, deacetylated desulfated keratan sulfate, deacetylated desulfated keratosulfate, esterified desulfated heparin, chitosan 1, chitosan 2, and combinations thereof.

39. The method of claim 36, wherein the negatively charged species of glycosaminoglycan is sodium hyaluronate and the positively charged species of glycosaminoglycan derivative is selected from the group consisting of chitosan 1 and chitosan 2.

40. The method of claim 36, wherein the negatively charged species of collagen derivative is succinylated collagen.

41. The method of claim 40, wherein the positively charged species of glycosaminoglycan derivative is selected from the group consisting of chitosan 1 and chitosan 2.

42. The method of claim 36, wherein the positively charged species of collagen derivative is methylated collagen.

43. The method of claim 42, wherein the negatively charged species of glycosaminoglycan is sodium hyaluronate.

44. A method for administering a pharmaceutically active agent to a mammal comprising:
injecting a liquid composition comprising one or more species of glycosaminoglycan derivative, which exhibits both positive and negative charges at a pH within a range from about 6 to about 8.5, in admixture with a therapeutically effective amount of a pharmaceutically active agent; and
permitting the liquid composition to form a gel *in situ* by adjustment of the liquid composition pH to within the pH range from about 6 to about 8.5.

45. The method of claim 44, wherein the glycosaminoglycan derivative is selected from the group consisting of deacetylated hyaluronic acid, deacetylated desulfated chondroitin sulfates A, deacetylated desulfated dermatan sulfate, deacetylated desulfated chondroitin sulfate C, desulfated heparin, and combinations thereof.

46. The method of claim 44, wherein the glycosaminoglycan derivative is deacetylated hyaluronic acid.

47. The method of claim 44, wherein the pharmaceutically active agent is selected from the group consisting of a cytokine, a growth factor, a chemotherapeutic agent, and an antibiotic.

48. A method for administering a pharmaceutically active agent to a mammal comprising:
injecting a liquid composition comprising one or more negatively charged species of glycosaminoglycan, glycosaminoglycan derivative or collagen derivative, and one or more positively charged species of glycosaminoglycan derivative or collagen derivative, in admixture with a therapeutically effective amount of a pharmaceutically active agent; and
permitting the liquid composition to form a gel *in situ* by adjustment of the liquid pH to within the pH range from about 6 to about 8.5.

49. The method of claim 48, wherein the negatively charged species of glycosaminoglycan or glycosaminoglycan derivative is selected from the group consisting of sodium hyaluronate, keratan sulfate, keratosulfate, sodium chondroitin sulfate A, sodium dermatan sulfate B, sodium chondroitin sulfate C, heparin, esterified chondroitin sulfate C, esterified heparin, and combinations thereof.

50. The method of claim 48, wherein the positively charged species of glycosaminoglycan derivative is selected from the group consisting of esterified deacetylated hyaluronic acid, esterified deacetylated desulfated chondroitin sulfate A, esterified deacetylated desulfated chondroitin sulfate C, deacetylated desulfated keratan sulfate, deacetylated desulfated keratosulfate, esterified desulfated heparin, chitosan 1, chitosan 2, and combinations thereof.

51. The method of claim 48, wherein the negatively charged species of glycosaminoglycan is sodium hyaluronate and the positively charged species of glycosaminoglycan derivative is selected from the group consisting of chitosan 1 and chitosan 2.

52. The method of claim 48, wherein the negatively charged species of collagen derivative is succinylated collagen.

53. The method of claim 52, wherein the positively charged species of glycosaminoglycan derivative is selected from the group consisting of chitosan 1 and chitosan 2.

54. The method of claim 48, wherein the positively charged species of collagen derivative is methylated collagen.

55. The method of claim 54, wherein the negatively charged species of glycosaminoglycan is sodium hyaluronate.

56. The method of claim 55, wherein the pharmaceutically active agent is selected from the group consisting of a cytokine, a growth factor, a chemotherapeutic agent, and an antibiotic.
